# EUROPEAN PATENT APPLICATION

(11) **EP 4 783 185 A1**
(43) Date of publication of application: **29.07.2026**
(21) Application number: 25154498.7
(22) Date of filing: 28.01.2025
(51) Int. Cl.: G16H 20/70, A61M 21/00

(54) **VIRTUAL REALITY THERAPY SYSTEM, METHOD AND COMPUTER PROGRAM**

(71) Applicant: Lab E GmbH, 73728 Esslingen (DE)
(72) Inventor: SCHOLLER, Patrik, 73728 Esslingen (DE); EPPLE, Carola Helen, 73728 Esslingen (DE)
(74) Representative: Eisenführ Speiser

(57) **Abstract**

The present invention relates to a computer-implemented method (100) for virtual reality assisted therapy and/or differential diagnosis, a corresponding apparatus, system and use. The method comprises: exposing (102) a user to virtual reality content via a virtual reality headset (202) during a exposure session; capturing (104), via sensors, data of the user related to the exposure session virtual reality content; and processing (106) the data using a computer-implemented algorithm to adjust the virtual reality content based on predefined therapeutic objectives.

## Description

The present disclosure relates to a system, method, and apparatus for virtual reality assisted therapy and differential diagnosis. Specifically, the present disclosure pertains to the use of virtual reality headsets, biometric sensors, and computer-implemented algorithms to dynamically adjust therapeutic content and optimize therapeutic outcomes based on user-specific data.

The present invention is primarily used to assist the treatment of psychological health disorders. In particular, the present invention is used to assist the treatment of anxiety disorders. However, it is not limited to this condition and it can be applied to other conditions such as, without being limited, addiction, stress, compulsion, tension, depression, ADHD, trauma, etc., depending on the virtual reality content provided by the system. Preferentially, the present invention applies to all psychological health disorders that can be diagnosed and assessed using clinically verified questionnaires.

Anxiety disorders, including phobias, are among the most prevalent mental health conditions, characterized by intense and irrational fears triggered by specific objects or situations. These fears often lead to avoidance behaviors that significantly impact an individual's quality of life. Psychotherapeutic treatments, such as cognitive behavioral therapy (CBT) and exposure therapy, have been widely recognized for their effectiveness in managing these disorders. Exposure therapy, in particular, is recommended as the gold standard in the S3 guideline for treating anxiety disorders. This approach involves gradual and controlled exposure to anxiety-inducing stimuli, either in real-world settings (in-vivo) or through mental visualization (in-sensu), to help patients disassociate the feared stimuli from anticipated negative outcomes, ultimately reducing anxiety responses.

Despite its proven efficacy, traditional exposure therapy poses several challenges, including substantial time, resource, and logistical constraints. For example, the preparation and execution of real-world scenarios for therapeutic purposes, such as providing physical stimuli for phobia treatment, can be both cost- and labor-intensive. One example relates to social phobia in school situations, which is both logistically and practically very difficult to reproduce in reality.

Virtual Reality Exposure Therapy (VRET) enables patients to be confronted with relevant anxiety-inducing stimuli in a safe and controlled virtual setting. By simulating real-world scenarios, VRET facilitates gradual and systematic exposure, a cornerstone of traditional exposure therapy, with enhanced precision and adaptability to therapy progress. Clinical studies, including meta-analyses by Wechsler et al. (2019) and Carl et al. (2019), confirm that VRET achieves comparable outcomes to traditional exposure therapy while addressing many logistical barriers.

Furthermore, VR-assisted cognitive behavioral therapy (VRCBT) has emerged as an effective approach to address anxiety and related conditions. Integrating VR technology with CBT frameworks enables real-time monitoring of patient responses and dynamic adaptation of therapeutic content. This approach likely accelerates therapeutic outcomes and enhances long-term effectiveness through manageable virtual follow-up exposures.

However, current implementations of VRET and also VRCBT remain largely confined to professional therapy settings, limiting their accessibility and scalability.

It was therefore an objective of the present invention to overcome the limitations in accessibility and scalability of existing VRET and VRCBT frameworks and solutions. It was a further objective of the present invention to provide methods, systems and apparatuses that improve the clinical results when treating conditions such as anxiety disorders with VRET and VRCBT.

A first aspect of the invention provides a computer-implemented method for virtual reality assisted therapy and/or differential diagnosis, comprising: -exposing a user to virtual reality content via a virtual reality headset during an exposure session; -capturing, via sensors, data of the user related to the exposure session virtual reality content; -processing the data using a computer-implemented algorithm to adjust the virtual reality content based on predefined therapeutic objectives.

The claim describes a computer-implemented method designed for use in virtual reality-assisted therapy and differential diagnosis. The method involves several key components and steps. The aim of the present invention is to expose users to adequate virtual reality content so that achieving a therapeutic objective is supported. Treatment and therapy is not achieved by the solutions according to the present invention alone, but the treatment and diagnosis of a medical professional, such as a psychotherapeutic, is assisted and the outcome of the therapy improved through the concepts and aspects of the present invention, as will be detailed below.

The gist of the present invention is to use feedback from the user, i.e., data of the user, to evaluate whether or not the virtual reality content the user is exposed to is adequate based on the therapeutic objectives, and, based on the evaluation result, to adjust the content if appropriate. More specifically, the method in particular analyzes the user's progress in view of the therapeutic objectives and determines whether or not the virtual reality content the user is exposed to is appropriate for the user's progress or not. For instance, it may not be appropriate if it is insufficient to trigger sufficient user reaction, i.e., no therapeutic progress is achieved. On the other end, it may not be appropriate if it triggers excessive reaction that may be difficult for the user to control, i.e., overwhelms the user.

Initially, a user is exposed to virtual reality content through, for instance, a virtual reality headset during what is referred to as an exposure session. This step is crucial as it immerses the user in a controlled virtual environment tailored to therapeutic or diagnostic purposes.

During or after this session, sensors capture data related to the user's interaction with the virtual reality content. This data can include physiological responses, movements, or other relevant metrics that provide insight into the user's state or reactions. The data can also include written or spoken responses or feedback requested from the user.

The captured data is then processed using a computer-implemented algorithm. The purpose of this processing is to adjust the virtual reality content in real-time or for future sessions, based on predefined therapeutic objectives. These objectives are likely determined by healthcare professionals and are intended to achieve specific therapeutic outcomes or to aid in differential diagnosis. Additionally or alternatively, the users themselves can add or define goals and objectives, for instance in case of stress or after receiving clearance from the healthcare professional in their own aftercare, enabling patient autonomy and patient sovereignty.

The advantages of this method include providing a personalized and adaptive therapeutic experience, enhancing the effectiveness of therapy or diagnosis by tailoring the virtual environment to the user's needs and responses in the course of therapy.

In a first implementation of the method according to the first aspect, the predefined therapeutic objectives are determined based on responses from the user to an initial questionnaire before starting the therapy session, the determination of the predefined therapeutic objectives includes in particular: -obtaining information indicating at least one of a frequency and an intensity of a user experience related to a psychological health disorder on a scale such as a Liebowitz Social Anxiety Scale (LSAS) or Likert-type scale, -scoring the obtained information to obtain a total score indicative of the severity of psychological health disorders, e.g. psychological health disorder symptoms and/or one or more separate subscores for fear, anxiety and avoidance, and -defining the therapeutic objectives to guide treatment decisions and evaluate treatment effectiveness based on the total score and/or the one or more subscores.

Psychological health disorders encompass a broad range of conditions that affect an individual's emotional well-being, mental health, and behavioral patterns. These disorders, which may arise from biological, psychological, or environmental factors, influence how individuals think, feel, and behave in various aspects of life.

Examples include without being limited: a) anxiety disorders, characterized by excessive fear, worry, or nervousness, such as generalized anxiety disorder, social anxiety disorder, panic disorder, and specific phobias; b) mood disorders, including major depressive disorder, bipolar disorder, and dysthymia, primarily affect emotional states. c) substance use disorders, such as alcohol use disorder, opioid use disorder, and stimulant use disorder, involve the misuse or dependence on substances like alcohol, opioids, stimulants, cannabis, or nicotine; d) eating disorders, such as anorexia nervosa, bulimia nervosa, binge-eating disorder, and avoidant/restrictive food intake disorder, are marked by abnormal eating habits and concerns about body weight or shape; e) obsessive-compulsive and related disorders include obsessive-compulsive disorder, hoarding disorder, and body dysmorphic disorder, which involve repetitive behaviors or intrusive thoughts; f) post-traumatic stress disorder (PTSD) and other trauma-related disorders develop following exposure to traumatic events, causing symptoms like flashbacks, hyperarousal, and avoidance behaviors; g) attention-deficit/hyperactivity disorder (ADHD), which is a neurodevelopmental disorder characterized by inattention, hyperactivity, and impulsivity; h) autism spectrum disorder (ASD), which involves challenges in social interaction, communication, and repetitive behaviors, often accompanied by sensory sensitivities; i) psychotic disorders, such as schizophrenia and schizoaffective disorder, distort perceptions of reality and may include hallucinations, delusions, and disorganized thinking; j) somatic symptom and related disorders, such as somatic symptom disorder and illness anxiety disorder, involve physical symptoms without a medical cause or with excessive concern about health; k) impulse-control disorders, including intermittent explosive disorder, kleptomania, and pyromania, are marked by difficulty controlling impulses; I) behavioral addictions, such as gambling disorder, internet gaming disorder, and compulsive shopping, involve non-substance-related compulsive behaviors; m) sleep-wake disorders, such as insomnia disorder, hypersomnia, and narcolepsy, affect sleep patterns and behaviors; n) personality disorders, including borderline personality disorder and antisocial personality disorder, involve long-term patterns of behavior and inner experiences that deviate from cultural norms; o) developmental disorders, such as intellectual disability and speech or language disorders, arise during developmental periods and affect cognitive or emotional development; p) neurocognitive disorders, including dementia and mild cognitive impairment, which are characterized by cognitive decline.

Psychological health disorders often co-occur and present with varied manifestations across individuals. Treatments typically aim to address underlying causes, reduce symptoms, and improve quality of life through approaches such as psychotherapy, pharmacological therapies, lifestyle changes, and holistic interventions. The present invention using real-time assisted virtual reality exposure to assist the therapy assists in achieving therapeutic objectives for a broad range of these and other examples of psychological health disorders incl. behavioral disorders.

As an example, the questionnaire for social phobia is described, wherein as discussed also other clinical conditions can be evaluated accordingly using adopted questionnaires as known to the skilled people.

The initial or anamnesis questionnaire for a given psychological health disorder assessment serves as a pivotal diagnostic tool designed to evaluate the specific experiences, triggers, and symptoms associated with one specific or different psychological health disorders, such as social anxiety. By systematically collecting data through structured and evidence-based inquiries, the questionnaire ensures a thorough understanding of the patient's condition. This process facilitates accurate diagnosis and lays the groundwork for personalized treatment interventions.

The primary objective of the questionnaire is to explore the wide spectrum of psychological disorder symptoms. These symptoms often manifest as psychological, physical, and behavioral reactions in various social contexts. The design incorporates a comprehensive array of questions to uncover the nuanced ways in which the psychological disorder affects individuals. By capturing data on specific scenarios and their impact, the questionnaire allows for an in-depth analysis of symptom severity and triggers.

The questionnaire for example examines a broad range of social situations that commonly elicit anxiety or trigger other psychological disorder conditions, ensuring that the diagnostic process accounts for the diverse nature of different health disorder conditions. It uses a structured response format, typically relying on Likert scales, where respondents rate the intensity or frequency of their experiences. These scales allow for the easy quantification of subjective experiences, enabling clinicians to compare results over time and across populations. Additionally, the questions are tailored to reflect real-world scenarios, such as public speaking, interpersonal interactions, and participation in group activities, ensuring the collected data is directly applicable to everyday life.

In the following, social anxiety is described as one example of a psychological disorder condition that is relevant for the present invention. Likewise, other examples of psychological disorders can be addressed with adequate questionnaires in the same way. The questionnaire systematically addresses the core dimensions of social anxiety, including the psychological, physical, and behavioral components. Key areas of inquiry include:
1. Fear of performance and authority, such as anxiety when speaking in public or interacting with authority figures.
2. Interpersonal interactions, including discomfort in conversations with strangers or individuals of the opposite sex.
3. Observation by others, such as fear of being watched while eating, writing, or performing tasks.
4. Avoidance behaviors, including tendencies to avoid social events or leave them prematurely.
5. Physical symptoms, such as a racing heart, sweating, or shaking in social situations.
6. Fear of rejection or embarrassment, including worry about making mistakes or being criticized in social contexts.
7. Difficulty with assertiveness, such as trouble expressing disagreement or standing up for oneself.
8. Romantic and intimate interactions, including anxiety about asking someone on a date or initiating romantic conversations.

The scoring methodology quantifies the severity of social anxiety symptoms, providing clear metrics for analysis. Respondents rate each question on a scale, such as 0 to 4, indicating the frequency or intensity of their experiences. Total scores are calculated by summing the ratings for all items, with higher scores indicating greater severity. Some questionnaires, such as the Liebowitz Social Anxiety Scale (LSAS), provide separate subscores for fear and avoidance dimensions, offering deeper insights into the interplay between these aspects.

Thresholds are used to categorize severity levels. For example, a score of 19 or higher on the Social Phobia Inventory (SPIN) suggests the presence of social anxiety disorder, while scores above 60 on the LSAS indicate generalized social anxiety disorder. In addition, some tools, such as the Social Anxiety Questionnaire for Adults (SAQ), assess multiple dimensions of social anxiety, such as fear of strangers and public speaking, for a more nuanced understanding.

The results of the questionnaire guide clinical decisions in several ways. It acts as a preliminary screening tool, identifies individuals who may require further evaluation and treatment, informs the selection of therapeutic approaches, and allows for the monitoring of symptom changes over time. Regular administration enables clinicians to track progress and evaluate treatment effectiveness.

In the present invention, the results are preferentially used to define the therapeutic objectives that include personalized exposure scenarios targeting the specific triggers identified during the assessment. In particular, the results preferentially include both a present indicator characterizing the present status and a target indicator characterizing a therapy goal in which the user's condition is improved.

The anamnesis questionnaire is a vital component of the diagnostic and therapeutic process for psychological disorders. Its structured design, broad coverage, and actionable insights enable clinicians to identify and address psychological disorder symptoms effectively. When integrated into the framework according to the present invention, this tool empowers clinicians to deliver personalized, evidence-based care that improves patient outcomes.

In other words, this embodiment introduces specific mechanisms of communication between components by detailing how predefined therapeutic objectives are determined based on user responses to an initial questionnaire administered before the exposure session begins. This process involves obtaining information that indicates the frequency and intensity of a user's experience using a scale such as a Likert-type scale. The obtained information is then scored to produce a total score that is indicative of the severity of psychological disorder symptoms. These scores are subsequently used to define the therapeutic objectives, which serve to guide treatment decisions and evaluate the effectiveness of the treatment. The new features brought by this dependent claim include the integration of a user-specific initial assessment that tailors the virtual reality therapy to the individual's specific needs. By quantifying the user's symptoms through a standardized scale and scoring system, the method ensures that the therapeutic content is personalized and dynamically adjusted to address the user's particular issues. This approach enhances the precision and effectiveness of the therapy by providing a structured framework for setting therapeutic goals and measuring progress.

The in some embodiments preferred use of a Likert-type scale for initial assessment introduces a quantitative method for evaluating the user's condition, which can be critical for both diagnosing the severity of the psychological disorder such as social anxiety and for monitoring changes over time.

The scoring system that generates a total score and subscores allows for a nuanced understanding of the user's symptoms, enabling more targeted selection suitable exposure scenarios in the context of therapeutic intervention.

By defining therapeutic objectives based on these scores, the method ensures that the virtual reality content is not only relevant to the user's current state but also adaptable to changes in their condition, thereby optimizing the therapeutic process. This claim thus adds a layer of customization and responsiveness to the virtual reality assisted therapy, making it more effective and user-centric.

In a further implementation of the method according to the first aspect a collection of elements of virtual reality content is provided.

The method now includes providing a collection of virtual reality content elements, with each element classified within a multi-dimensional space comprising at least two dimensions. This classification allows for a more nuanced and structured organization of the virtual reality content, facilitating more precise adjustments based on therapeutic needs.

The computer-implemented algorithm is further detailed to include steps that employ predefined therapeutic objectives to obtain a progressively sorted ranking of these elements. This ranking system ensures that the user's exposure to virtual reality content can be dynamically adjusted in a manner that aligns with the therapeutic goals, providing a tailored experience for the user.

The algorithm processes the captured data to derive a therapy progress decision variable, which serves as an indicator of the user's progress within the therapeutic framework. This decision variable is then used to select an appropriate element from the progressively sorted ranking, ensuring that the virtual reality content is adjusted in a manner that is responsive to the user's current state and progress.

The new features introduced by this preferred embodiment bring a higher level of customization and adaptability to the virtual reality assisted therapy and/or differential diagnosis method. By incorporating a multi-dimensional classification of content elements and a progressively sorted ranking system, the method can more effectively address the specific therapeutic needs of the user. The use of a therapy progress decision variable further enhances the method's ability to provide real-time adjustments to the virtual reality content, ensuring that the therapy remains relevant and effective as the user progresses. This level of detail and specificity in the communication between components not only improves the overall functionality of the method but also provides a more robust framework for achieving the predefined therapeutic objectives.

In a further implementation of the method according to the first aspect, the therapy progress decision variable indicates either of "up", "down" and "horizontal variation on the same level" along the progressively sorted ranking of the elements.

The dependent claim introduces a specific mechanism for determining the progression of therapy through the use of a decision variable that indicates the direction of therapy progress. This decision variable can take on values such as "up," "down," or "horizontal variation on the same level," which correspond to the user's movement along a progressively sorted ranking of elements within the virtual reality therapy framework. The communication between components in this context involves the sensors capturing data related to the user's interaction with the virtual reality content during the exposure session. This data is then processed by a computer-implemented algorithm, which is a feature of the independent claim, to evaluate the user's progress in therapy. The algorithm assesses the captured data against predefined therapeutic objectives and determines the appropriate direction of therapy progress, as indicated by the decision variable. The introduction of this decision variable as a feature brings a structured and quantifiable method for evaluating and adjusting the therapy process. By providing distinct indicators of progress, the method allows for a more nuanced and dynamic adjustment of the virtual reality content, tailoring it to the user's specific therapeutic needs. The "up" indication suggests that the user is progressing positively and may be ready for more advanced therapeutic content, while the "down" indication implies a need to revisit simpler or foundational elements. The "horizontal variation on the same level" suggests maintaining the current level of difficulty or focus, allowing for consolidation of progress or exploration of different therapeutic elements at the same level of complexity. This structured approach to therapy progression enhances the adaptability and effectiveness of the virtual reality assisted therapy by providing clear, actionable feedback on the user's progress. It allows therapists to make informed decisions about the direction and intensity of the therapy, ensuring that it remains aligned with the user's evolving needs and therapeutic goals.

In a further implementation of the method according to the first aspect, the at least two dimensions include at least two of: a) intensity of the element, b) degree of motion, c) tension build-up, d) noise level, and e) mood level. Preferentially, the dimensions include all five categories.

The current dependent claim introduces specific mechanisms of communication between components by detailing the dimensions that can be adjusted within the virtual reality content. These dimensions, which are based on scientifically evaluated studies, include the intensity of the element, the degree of motion, tension build-up, noise level, and mood.

Each of these dimensions represents a different clinically relevant aspect of the virtual reality experience that can be manipulated to tailor the therapy to the user's needs, e.g. increasing tension in social phobia exposures by showing people coming closer and closer, whereas, in addiction, changing mood from neutral to more and more positive while increasing noise level.

The intensity of the element allows for customization based on the severity or specifics of the condition being treated, ensuring that the virtual reality content is appropriately challenging or soothing.

The degree of motion of the virtual reality content can be adjusted to either increase or decrease the physical engagement required from the user, which can be particularly useful for conditions where physical activity is a factor and plays a therapeutic role.

In one implementation, the motion adjustment refers specifically to the characteristics of the virtual reality environment itself, such as whether the visual content is static (e.g., a serene forest scene), gently dynamic (e.g., a peaceful walk through the woods), moderately dynamic (e.g., a moving car ride), or highly dynamic and jarring (e.g., a suspension bridge swaying over a canyon or the turbulence experienced during a flight). These varying levels of visual motion can be tailored to align with the user's emotional and physical state, creating a scalable therapeutic approach.

In another embodiment, the virtual reality system integrates a more interactive layer, where the system, in particular implemented using an artificial intelligence including a chatbot, not only selects and adapts the motion of the virtual reality scenes but also actively guides the user through additional activities to enhance emotional and physical engagement. For example, while immersed in a calming forest scene, the bot could recommend controlled breathing exercises to manage anxiety or suggest physical activities such as squats or stretches to promote relaxation and physical grounding. This integrated approach bridges passive virtual reality experiences with active participation, creating a holistic therapeutic method.

Alternatively, the system may emphasize a passive observational experience, where the user is exposed solely to the adjusted motion of the virtual reality content. Here, the focus remains on stimulating emotional responses and enabling the user to practice behavioral strategies to cope with these responses. For example, in a static scene such as a forest, the user may focus on mindfulness or relaxation techniques, while a more dynamic scene, such as a suspension bridge swaying in the wind, could help the user confront and manage feelings of fear or unease in a controlled environment.

In a further refinement, the virtual reality content and associated physical activities could be dynamically adjusted in real time based on biometric feedback as an addition or alternative to articulated spoken conversations or feedback with the bot. For instance, sensors monitoring heart rate, breathing patterns, or muscle tension could guide the system to reduce the motion of the content or suggest calming exercises if elevated stress levels are detected. Conversely, if the user's engagement or focus appears low, the system might increase the intensity of the virtual reality motion (e.g., show a different scene) or recommend more vigorous physical activities.

A related implementation could limit the bot's role to providing motion-based scenarios without integrating physical activity guidance. This embodiment focuses solely on the visual and emotional aspects of the therapy, ensuring the system remains flexible and adaptable for users who may not be able to participate in physical activities due to mobility issues or other constraints.

Tension build-up is generally dependent on the condition and differs, for instance, between social anxiety, ADHS or stress disorders. It refers to the gradual increase in stress or challenge within the virtual reality scenario, which can be used to help users build resilience or cope with condition-inducing situations.

Noise level can be controlled to either simulate real-world environments more accurately or to provide a more controlled and less distracting setting.

Mood adjustments can alter the emotional tone of the virtual reality content, making it more uplifting or calming or even actively depressing as needed.

The introduction of a multi-dimensional space for classifying the elements of the virtual reality content allows to independently adjust the exposure experience along plural dimensions to create a highly customized therapeutic experience. This multi-dimensional approach allows for a more nuanced and effective therapy, as it can be tailored to the specific needs and responses of the user.

Additionally, the embodiment hints at the use of artificial intelligence to generate relevant elements or video scenes based on typical scores from questionnaires, which suggests a level of automation and personalization in the creation of the virtual reality content. This Al-driven approach allows to streamline the process of developing therapy sessions, ensuring that they are both relevant and effective for the user's specific condition.

In another aspect, the invention therefore provides an automatic VR video script that creates or selects scenes relevant to a specific type of social phobia and adapts them for therapy. This aspect underscores the innovation in using technology to enhance therapeutic outcomes.

In a further implementation of the method according to the first aspect, each of the dimensions of the element is classified as at least one of four levels.

The dependent claim introduces a classification mechanism for the dimensions of the element within the virtual reality assisted therapy and/or differential diagnosis method. Specifically, it stipulates that each dimension of the element is classified into one of four levels. This classification mechanism serves as a means of organizing and categorizing the collection of elements of virtual reality content.

By classifying each dimension into one of four levels, the method can more precisely select the element of the virtual reality content to meet current user's progress in view of the predefined therapeutic objectives.

The new feature introduced by this specific embodiment is the structured classification of dimensions, which enhances the granularity and specificity of the selected element of virtual reality content. This allows for a more nuanced and effective therapeutic intervention or differential diagnosis.

The combination of dimensions and levels creates a multi-dimensional space with certain discrete points at which the several elements of virtual reality content can be classified. The method allows to select the most adequate element in view of the user's therapy process and thereby to support the user's progress.

It is worth noting that it is not necessary to completely populate each point in the multi-dimensional space, i.e., it is not necessary to prepare and provide an element of virtual reality content for each combination of dimension and intensity. By not populating each point in the multi-dimensional space with content, the method avoids undue work and effort for content that is not relevant in actual therapy plans. Necessary storage space can be saved and required resources reduced. This selective approach ensures that the virtual reality content remains manageable and targeted, improving the overall efficacy of the treatment or diagnostic process.

The classification into four levels provides a clear framework for evaluating and adjusting the virtual reality content, making the system more robust and adaptable to different therapeutic scenarios. This feature also facilitates better tracking and analysis of the user's progress, as the classified data can be systematically reviewed and compared over time.

In a further implementation of the method according to the first aspect, the progressively sorted ranking is obtained by applying a bias in the multi-dimensional space based on the predefined therapeutic objectives.

For example, in the context of social phobia in a school setting:
Initially, the situation may be moderate and neutral, such as before a presentation begins, where only a few children are still paying attention, the class remains quiet, and the teacher does not exhibit active judgment.

Gradually, the scenario shifts, with children beginning to talk among themselves, ceasing to listen, and some displaying judgmental expressions. The intensity and noise level increase, which may lead to an amplified emotional response and heightened anxiety.

In this example of social phobia in a school setting, intensity and noise level may have the highest bias. In other examples, the therapeutic objectives may bias other dimensions.

The dependent claim introduces a specific mechanism for obtaining a progressively sorted ranking by applying a bias in the multi-dimensional space based on predefined therapeutic objectives. This bias is based on the predefined therapeutic objectives, which means that the algorithm is not merely processing data in a neutral manner but is actively incorporating therapeutic goals into its calculations, in the case of the example, it is about judging and ignorance of self, a special type of social phobia. In other cases, it might be the fear of proximity of people increasing (from far to close to touching) and their mood (from friendly to aggressive). This allows for a more tailored and effective therapeutic experience, as the virtual reality content can be adjusted in a way that progressively aligns with the therapeutic objectives. For example, if the therapeutic objective is to reduce anxiety, the algorithm might apply a bias that prioritizes calming and soothing virtual reality content, progressively ranking this type of content higher in the multi-dimensional space. Conversely, if the objective is to confront and overcome specific fears, the bias might prioritize exposure to those fears in a controlled and gradual manner. This feature enhances the adaptability and precision of the virtual reality assisted therapy, making it more responsive to the individual needs of the user. By incorporating a bias based on therapeutic objectives, the system can provide a more personalized and effective therapeutic intervention, potentially leading to better outcomes for the user.

In a further implementation of the method according to the first aspect, at least one of the elements comprises a decision point at which one of two continuing elements associated with a different point in the multi-dimensional space, respectively, can be taken, the selection of the respective continuing element is performed by processing the data of the user.

The dependent claim introduces a specific mechanism of communication between components by incorporating a decision point within at least one of the elements. This decision point allows for the selection between two continuing elements, each associated with a different point in the multi-dimensional space.

The selection process is performed by processing the data of the user. This new feature adds a layer of interactivity and adaptability to the virtual reality assisted therapy and/or differential diagnosis method described in the independent claim. By introducing decision points, the system can dynamically alter the course of the virtual reality content based on real-time data captured from the user. This enhances the therapeutic or diagnostic process by making it more responsive to the user's immediate needs and reactions.

The decision points act as junctures where the system evaluates the user's data and determines the most appropriate path forward, thereby personalizing the experience. This can lead to more effective therapy sessions or more accurate differential diagnoses, as the system can tailor the virtual reality content to better align with the predefined therapeutic objectives.

In a further implementation of the method according to the first aspect, the selection of the respective continuing element is obtained by processing at least one of biofeedback from the user and a user response to a chatbot inquiry.

The dependent claim introduces specific mechanisms of communication between components by specifying that the selection of the respective continuing element is obtained by processing at least one of biofeedback from the user and a user response to a chatbot inquiry. This claim enhances the independent claim by detailing how the system interacts with the user and gathers data to refine the virtual reality content.

The inclusion of biofeedback as a mechanism means that the system can monitor physiological signals such as heart rate, skin conductance, or brain activity, which provides real-time, objective data about the user's physical and emotional state during the exposure session.

This real-time monitoring allows the system to make immediate adjustments to the virtual reality content, ensuring that the therapeutic objectives are met more effectively.

On the other hand, the user response to a chatbot inquiry introduces an interactive element where the user can provide subjective feedback through a conversational interface. This could involve the user answering questions about their comfort level, emotional state, or specific reactions to the virtual reality content.

By incorporating both biofeedback and user responses to chatbot inquiries, the system gains a comprehensive understanding of the user's condition from both objective physiological data and subjective self-reports. This dual approach allows for a more nuanced and personalized adjustment of the virtual reality content, thereby enhancing the therapeutic or diagnostic efficacy of the method. This integration of biofeedback and chatbot interactions represents a significant advancement in the field of virtual reality assisted therapy and differential diagnosis, providing a more situative and responsive approach to user care.

In a further implementation of the method according to the first aspect, the adjusting the virtual reality content is performed to maximize the difficulty along the progressively sorted ranking compatible with the user's therapy progress based on the user data.

The new feature introduced by this dependent claim is the dynamic adjustment of the difficulty level of the virtual reality content based on the user's therapy progress. This ensures that the therapy remains challenging and engaging for the user, promoting continuous improvement and adaptation to the user's evolving capabilities. By progressively increasing the difficulty, the method aims to optimize the therapeutic outcomes by providing a tailored and adaptive therapy experience. The dependent claim thus introduces a sophisticated mechanism for tailoring the therapy to the user's individual progress, leveraging the capabilities of virtual reality technology and data-driven algorithms to enhance therapeutic outcomes.

In a further implementation of the method according to the first aspect, the capturing data of the user comprises obtaining direct feedback after the exposure session.

This preferred embodiment introduces a specific mechanism for capturing data of the user, which comprises obtaining direct feedback after the exposure session. This feature brings a new dimension to the virtual reality assisted therapy and/or differential diagnosis method by incorporating a post-session feedback mechanism. The direct feedback can be collected through various means such as verbal responses, written questionnaires, or interactive digital interfaces, which allows for a more comprehensive understanding of the user's experience and reactions to the virtual reality content. This feedback mechanism serves as an additional layer of data that can be used to refine and adjust the virtual reality content more effectively based on the user's subjective experience. The inclusion of direct feedback enhances the therapeutic objectives by providing real-time insights into the user's mental and emotional state following the exposure session. This can lead to more personalized and adaptive therapy sessions, as the feedback can be immediately processed by the computer-implemented algorithm to make necessary adjustments to the virtual reality content. The communication between components in this context involves the sensors capturing the initial data during the exposure session, followed by the collection of direct feedback after the session, which is then processed by the algorithm. This sequential flow of data capture and processing ensures that the virtual reality content remains aligned with the predefined therapeutic objectives while also being responsive to the user's immediate feedback. The new feature of obtaining direct feedback after the exposure session adds a critical feedback loop to the method, enabling continuous improvement and customization of the therapy or diagnostic process.

In a further implementation of the method according to the first aspect, the capturing data of the user comprises at least one of: a) capturing biometric data of the user during the exposure session; b) interaction with a chatbot before, during and/or after the exposure session and c) receiving user responses to a questionnaire.

The embodiment introduces additional features by specifying the mechanisms for capturing data of the user. The claim outlines three specific mechanisms: capturing biometric data, interaction with a chatbot, and receiving user responses to a questionnaire. Each of these mechanisms - individually or collectively - enhances the method by providing different types of user data that can be used to tailor the virtual reality experience to the user's needs.

Capturing biometric data involves using sensors to collect physiological information such as eye movement, pupil size, facial expression, heart rate, blood pressure, skin conductance, muscle tension, breathing, movement, or other relevant metrics during the exposure session. This data can provide insights into the user's physical reactions to the virtual reality content, allowing the system to adjust the content in real-time to better align with therapeutic objectives.

The interaction with a chatbot introduces a conversational interface that can engage with the user before, during, and after the exposure session. This interaction can serve multiple purposes, such as gathering qualitative data about the user's emotional state, providing guidance or support, and facilitating a more personalized experience. The chatbot can be designed to use natural language processing algorithms to understand and respond to user inputs, creating a dynamic and interactive component of the therapy. The chatbot's algorithm or model could be trained to allow the patient to have more control over their experience, potentially enabling them to make decisions about the progression of the therapy rather than relying solely on Al-driven scene selection.

The third mechanism, receiving user responses to a questionnaire, provides a structured way to collect subjective data from the user. This can include self-reported measures of anxiety, comfort, or other psychological states that are relevant to the therapy or diagnosis. By integrating these diverse data sources, the method can offer a more comprehensive and adaptive therapeutic experience, potentially improving outcomes by aligning the virtual reality content with the user's specific therapeutic needs and objectives. The inclusion of these features demonstrates an effort to create a more holistic and user-centered approach to virtual reality assisted therapy and differential diagnosis.

This embodiment acknowledges the complexity of therapeutic processes and the need for human oversight, particularly from a psychologist, to address multifaceted issues such as social phobia. Therapy includes significantly more than exposure to virtual reality content, the systems and methods according to the invention assist in and improve the therapy outcome.

In a further implementation the method further comprises providing real-time guidance and feedback during an exposure session by a chatbot.

The chatbot functions as an intermediary, receiving data from the sensors that capture user responses and behaviors during the exposure session. The chatbot, leveraging this processed data, provides real-time feedback and guidance to the user, simulating the role of a therapist. This interaction ensures that the user receives immediate and contextually relevant support, enhancing the safety and efficacy of the therapy process.

The chatbot's real-time communication capability allows for dynamic adjustments to the virtual reality content, ensuring that the therapeutic objectives are met more effectively. Additionally, the chatbot's ability to replicate the role of a therapist means that users can receive personalized and adaptive support without the constant presence of a human therapist.

By providing real-time guidance and feedback, the chatbot ensures that the user remains engaged and motivated throughout the exposure session, which is crucial for the success of virtual reality assisted therapy. Furthermore, the chatbot can offer reassurance and instructions, helping to mitigate any discomfort or anxiety the user may experience during the session.

This real-time interaction is a significant advancement over traditional methods, where feedback is often delayed or requires the presence of a human therapist. The integration of a chatbot thus represents a substantial improvement in the method, providing a more interactive, responsive, and supportive therapeutic environment.

In a further implementation the method further comprises processing the data to identify unexpected patient reactions.

This embodiment incorporates an additional layer of analysis and response, thereby improving the system's adaptability and robustness. The processing of data to identify unexpected patient reactions involves the use of sophisticated algorithms that can detect anomalies or deviations from expected (e.g. trained) patterns of behavior during the virtual reality exposure session. This capability is crucial for ensuring that the therapy remains effective and safe, as it allows for real-time monitoring and assessment of the user's responses. By leveraging artificial intelligence to address these unexpected reactions, the system can dynamically adjust the virtual reality content or therapeutic approach to better suit the user's needs. This could involve modifying the intensity or nature of the virtual reality stimuli, providing additional guidance or support, or even pausing the session if necessary. The use of artificial intelligence in this context ensures that the system can handle a wide range of potential scenarios and patient responses, making it more versatile and capable of delivering personalized therapy. Furthermore, the integration of Al-driven responses helps in managing complex interactions that may arise during the therapy sessions, thereby enhancing the overall efficacy and safety of the treatment. This feature also underscores the importance of developing robust Al systems that can manage these interactions effectively. Additionally, the ability to identify and address unexpected patient reactions is a critical fail-safe mechanism that ensures the system can respond appropriately to unforeseen circumstances, thereby maintaining the integrity and safety of the therapy. This is particularly important in a self-administered VR therapy system, where the absence of a human therapist necessitates reliable automated responses to ensure patient safety. Overall, the new feature introduced in this embodiment significantly enhances the functionality and reliability of the virtual reality assisted therapy system, making it better equipped to handle the complexities and variabilities of patient responses during therapy sessions.

Developing a self-administered VR therapy system presents several challenges as mentioned above. These include ensuring that therapy scenarios dynamically adjust based on patient progress in coping with the exposure to a certain scenario level, developing robust Al systems capable of managing complex interactions, and implementing fail-safe mechanisms to address unexpected patient reactions. Comprehensive compliance with ethical, medical, and data protection regulations is essential to ensure the system's safety and efficacy. By addressing these challenges, the invention has the potential to transform psychological disorder treatment, offering significant clinical and health-economic benefits while providing accessibility for a large number of patients that currently have no access to cognitive behavioral therapy.

A second aspect of the invention provides an apparatus for virtual reality therapy, comprising: a virtual reality headset configured to display virtual reality content to a user; a plurality of sensors configured to capture biometric data of the user during the virtual reality therapy session; a processor configured to analyze the biometric data and dynamically adjust the virtual reality content to optimize therapeutic outcomes, wherein the processor is configured to perform the method according to any of the implementations.

The subject matter described involves an apparatus designed for virtual reality therapy. This apparatus includes a virtual reality headset that is designed to present virtual reality content to the user. Additionally, it incorporates multiple sensors that are capable of capturing the user's biometric data during the virtual reality therapy session. A processor is included, which is responsible for analyzing the captured biometric data and dynamically adjusting the virtual reality content to enhance therapeutic outcomes. The processor is also configured to execute the method or a preferred embodiment of the method according to the present invention.

The virtual reality headset is a device worn on the head that provides an immersive visual experience by displaying virtual reality content. The plurality of sensors refers to multiple devices that can detect and measure various physiological parameters of the user, such as a camera imaging the user, a heart rate, skin conductivity, or brain activity. The processor is a computational unit that processes the physiological parameters and data collected by the sensors and makes adjustments to the virtual reality content presented to the user based on this data to improve the effectiveness of the therapy.

A third aspect of the invention provides a computer program comprising instructions which, when executed by a processor, perform the method of any of the implementations.

In this context, a "computer program" refers to a set of coded instructions that a computer's processor can execute. "Instructions" are specific commands that direct the processor to perform certain operations. When these instructions are "executed by a processor," it means the processor is carrying out the commands as specified by the program. The "method of any of the implementations" refers to a series of steps or procedures outlined in the method according to the present invention, which the computer program is designed to implement. The advantage of this subject matter lies in its ability to automate the described method, ensuring consistent and efficient execution by leveraging the processing power of a computer. This can lead to increased accuracy, speed, and reliability in performing the specified method.

A fourth aspect of the invention provides a system for virtual reality therapy, comprising: a virtual reality display unit; a feedback control module configured to adjust visual and auditory stimuli based on user interaction and biometric feedback; a storage device for storing user-specific therapy data and training parameters, wherein the feedback control module is configured to operate conform the method of any of the implementations.

The subject matter described involves a system designed for virtual reality therapy. It includes a virtual reality display unit, which is a device that presents immersive visual content to the user, often through a headset or similar apparatus. The system also features a feedback control module. This module is responsible for adjusting the visual and auditory stimuli that the user experiences, based on both the user's interactions with the system and biometric feedback, which could include data such as heart rate, skin conductivity, or other physiological indicators derivable from, for instance, a camera image of the user. The system also incorporates a storage device that holds user-specific therapy data and training parameters, allowing for personalized therapy sessions tailored to the user's needs and progress.

The feedback control module is configured to operate according to a specific method according to the present invention, ensuring that it functions in a manner consistent with the broader system design. This configuration is particularly necessary for the continuous training of artificial intelligence, suggesting that the system may use machine learning techniques to improve therapy outcomes over time. The advantages of this system include providing a personalized and adaptive therapeutic experience, potentially enhancing the effectiveness of therapy by responding in real-time to the user's physiological and interactive cues.

A fifth aspect of the invention provides a device for facilitating virtual reality therapy, comprising: a display for rendering immersive virtual reality environments; a user interface for selecting therapy programs; a control unit configured to communicate with external health-monitoring devices and adapt therapy programs accordingly, wherein the control unit is configured to operate conform the method of any of the implementations.

The subject matter described is a device designed to facilitate virtual reality therapy. This device includes a display that renders immersive virtual reality environments, allowing users to be fully engaged in the therapy sessions. It also features a user interface that enables users to select various therapy programs tailored to their needs. This selection includes selecting specific elements of virtual reality content or a program variation such as "beginner" and "advanced".

In particular, selecting the therapy program might include including the chatbot as an avatar or mascot in the virtual reality content that corresponds to a recorded real world scene such that the user does not have a feeling of being alone. This may be referred to as a blended VR concept that includes artificial elements or components within a filmed real world VR scene.

External health-monitoring devices include mobile phones and smartwatches, without being limited to these. These devices can a) deliver relevant measurement data and/or b) activate reminders or the like.

A sixth aspect of the invention provides the use of a virtual reality headset and a biometric feedback system in the preparation of a therapeutic method for treating psychological disorders.

The claim describes the utilization of a virtual reality headset combined with a biometric feedback system to develop a therapeutic method aimed at treating psychological disorders. The integration of these two technologies allows for a more immersive and responsive therapeutic experience, potentially enhancing the effectiveness of treatments for psychological disorders by providing real-time feedback and creating controlled environments for exposure therapy. This approach can lead to more personalized and adaptive treatment plans, improving patient outcomes.

Further advantages and examples are described with reference to the enclosed drawings.
- Fig. 1: schematically illustrates a flowchart of the method according to the present invention.
- Fig. 2: schematically illustrates a system according to the present invention.
- Fig. 3: schematically illustrates a structure of the virtual reality content.
- Fig. 4: schematically illustrates a first example of a virtual reality content element.
- Fig. 5: schematically illustrates a second example of a virtual reality content element.

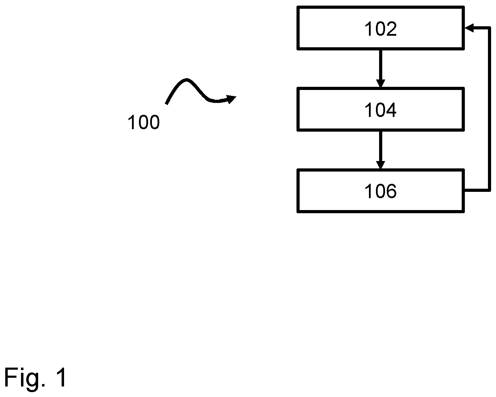

Fig. 1 schematically illustrates a flowchart of a computer-implemented method 100 for virtual reality assisted therapy and/or differential diagnosis according to the present invention.

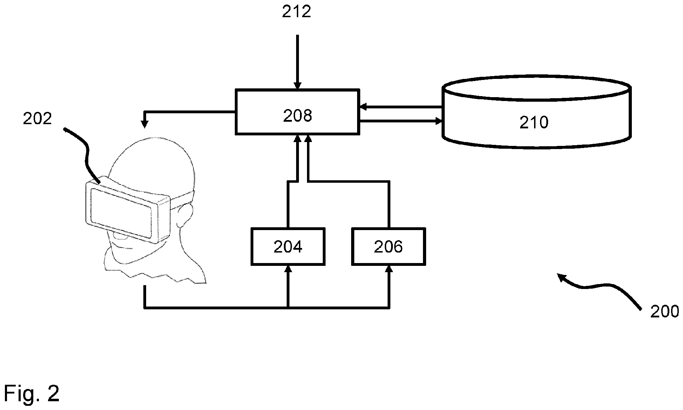

The method 100 includes a step 102 of exposing a user to virtual reality content via a virtual reality headset 202 (cf. Fig. 2) during an exposure session, a step 104 of capturing, via sensors, data of the user related to the exposure session virtual reality content, and a step 106 of processing the data using a computer-implemented algorithm to adjust the virtual reality content based on predefined therapeutic objectives. The method can then proceed with another exposure session in step 102 either directly or after some delay.

The gist of the present invention is to evaluate direct user's feedback to an exposure session to influence the current and future virtual reality content to which the user is and will be exposed.

Thereby, the virtual reality content is adjusted and tailored in order to support the user in the most efficient way towards achieving the therapeutic objectives.

Fig. 2 schematically and as an example illustrates a system 200. The system includes the virtual reality headset 202, a biometric feedback unit 204, a chat interface feedback unit 206, an evaluating unit 208 and a virtual reality content database 210 that will be described in the following with reference to the method of Fig. 1 in further detail.

In further detail, in step 102, a computer program such as a smartphone app or standalone application that operates the virtual reality headset 202 (see Fig. 2) and that is included in or in communication with evaluating unit 208 outputs virtual reality content via the display means integrated in virtual reality headset 202. The virtual reality content is obtained from virtual reality content database 210 that stores a plurality of elements of virtual reality content associated with plural attributes as described.

The evaluating unit 208 derives or obtains therapeutic objectives related to the user. The therapeutic objectives may be obtained from a physician or the like via interface 212. They can be either directly transmitted and received by evaluating unit 208 or entered manually by the user. Additionally or alternatively, the therapeutic objectives may be derived from user input to questionnaires as described.

The assessment to derive the therapeutic objectives or to obtain the therapeutic objectives is in the present invention a prerequisite to start immersion of virtual reality content. Thereby, wrong or harmful exposure may be avoided. Also, since the therapeutic objectives are defined prior to the exposure session, the application refers to them as predefined therapeutic objectives.

The evaluation module 208 can evaluate the user's progress towards the predefined therapeutic objectives and adjust the presented virtual reality content as appropriate. Thus, the evaluation module 208 may implement the step 102 of exposing the virtual reality content and the step 106 of processing the data as described in the following.

The evaluation module 208 obtains the virtual reality content element that corresponds most closely to the user's progress along the therapy route in view of plural dimensions of attributes of the video element. These dimensions in particular include intensity, degree of motion, tension build-up, noise level and mood.

The evaluation module 208 obtains feedback from at least one of the biometric feedback unit 204 and the chat interface feedback unit 206.

Biometric feedback unit 204 can obtain user's responses during the exposure sessions from sensors integrated into virtual reality headset 202 or observing the user by other means. Thereby, evaluation module 208 can derive the physiological response of the user to the exposure content and can determine whether or not the stimuli induced by the content are adequate or not.

Chat interface feedback unit 206 can in particular obtain written or spoken responses from the user to questions or otherwise directed conversations from a chat bot, in particular a large language model supported artificial intelligence chat bot. The chat bot can ask the user about his subjective feeling during the exposure session. In particular, the chat bot can ask the user for responses indicating a rating of his/her feeling and experience.

Depending on the feedback obtained from biometric feedback unit 204 and/or chat interface feedback unit 206, evaluation module 208 determines whether or not the user should progress along the therapy, stay at the current position (i.e., at the same virtual content element) or even move back because of too much response triggered in the user.

Preferentially, after every exposure session, the chat interface feedback unit 206 obtains direct feedback from the user including in particular the same feedback questions, such that the progress of the user can be accurately monitored.

Either or both of feedback unit 204 and feedback unit 206 can implement step 104 of capturing data of the user.

Virtual reality headset 202 is a device designed to immerse the user in a virtual environment for therapeutic purposes. The headset 202 comprises a display unit configured to render dynamic virtual reality content, creating a fully immersive experience. Preferentially, the headset includes integrated components such as motion sensors, gyroscopes, accelerometers, and eye-tracking systems to monitor user interactions and adjust the virtual content in response to head and eye movements. The headset 202 may also incorporate audio output devices, such as headphones or speakers, to provide synchronized auditory stimuli, enhancing the immersive experience.

Preferentially, the audio output device of the headset 202 is configured to output a chat interface from an Al chatbot. The headset 202 in this example further includes an audio input device such as a microphone to collect spoken response to the Al chatbot from the user. In other examples, audio input and audio output is implemented via other components remote from the headset, such as connected to a smartphone. The headset 202 can also display the questions from the chat bot via the display unit for the user to read.

The virtual reality content displayed on the headset 202 is generated and processed based on user-specific data and therapeutic objectives. This data may stem from various sources. In one embodiment, the headset retrieves locally stored content and data from an onboard processor or storage unit within the system itself. Alternatively, in another embodiment, the content and data may be streamed or downloaded from remote servers or cloud-based systems. These cloud-based systems enable dynamic updates and computational scalability, allowing the content to be tailored in real-time to the user's needs and therapy progress. Thus, one of, a plurality of or all of biometric feedback unit 204, chat interface feedback unit 206, evaluation module 208 and virtual reality content database 210 can be implemented locally on a computing device such as a smartphone, a desktop computer, a laptop computer, a stand-alone client or the headset 202 itself, and/or partially or fully on a remote or distributed computing device including but not limited to servers, cloud services and the like.

Preferentially, the virtual reality content is structured in database 210 and tagged using meta information aiming at different trigger situations. More preferred, the virtual reality content corresponds to real-world filmed scenes to provide the most realistic immersion to the user.

Furthermore, the headset 202 is configured to work in conjunction with not illustrated external components, such as biometric sensors and processing units, to enhance the user's experience. Chat response to chat bot enquiries and biometric data captured by external sensors, including heart rate, skin conductance, or pupil dilation, is processed by biometric feedback unit 204 and forwarded to evaluating unit 208. The adjustments to the virtual reality content from database 210 are based on this data. In cases where the processing occurs remotely, secure communication protocols are employed to transmit data between the headset and cloud servers, prioritizing both efficiency and data privacy.

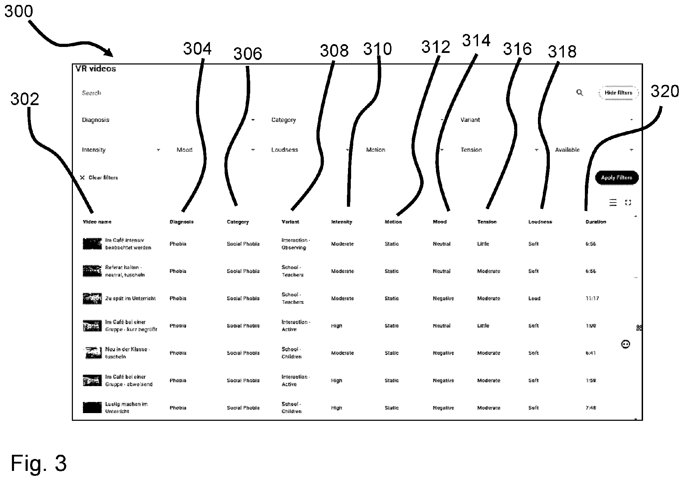

An example of a structured database 300 of virtual reality content is illustrated in Fig. 3. Each line in the list corresponds to one content element. In the first column 302, a name or indicator of the respective video is illustrated.

In the second column 304, third column 306, and fourth column 308 a three step specification of the condition variant the respective content addresses is specified, structured into diagnosis (e.g., Phobia), category (e.g. Social Phobia) and variant (e.g. "Interaction-Observing" or "School-Teachers").

The example framework organizes mental health conditions into categories, i.e., column 304, and variants, i.e. column 306, based on their triggers. Each category encompasses a range of detailed scenarios or stimuli to allow for precise diagnosis and tailored treatment.

An example of the classification into categories and variants is provided in the Examples section below.

Moreover, each virtual reality content is in this example categorized in five dimensions - intensity 310, motion 312, mood 314, tension 316, and loudness 318. An example of the classification in dimensions and levels and the respective meaning is also discussed in the Examples section below. Finally, a duration 320 of the content is provided.

In the present invention, the assessment of the initial questionnaire, for instance, allows the derivation of a category and variant for the mental health condition. Based on category and variant, the predefined therapeutic objective is derived.

For instance, the predefined therapeutic objective indicates an endpoint of the classification of the element of the virtual reality content in the multi-dimensional space defined by dimension and level, i.e., a "goal" for the user to arrive at when completing the therapy.

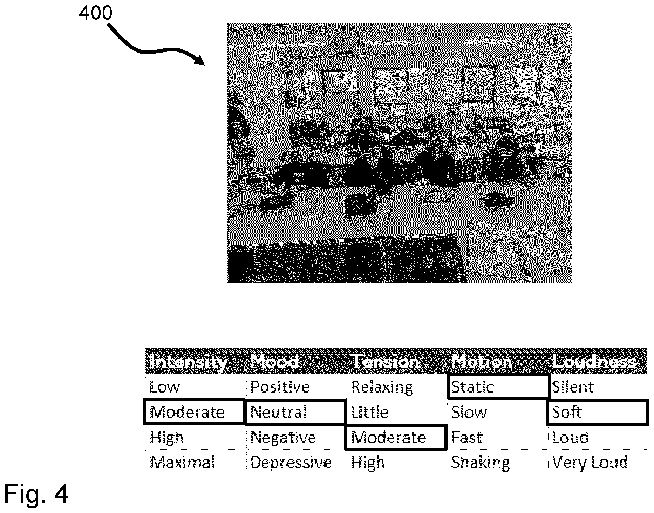

Fig. 4 illustrates an example virtual reality content 400 in the context of social phobia in a school setting. The scenario is classified as moderate intensity, neutral mood, such as before a presentation begins, where only a few children are still paying attention, the class remains quiet, and the teacher does not exhibit active judgment. Thus, the tension is classified as moderate, the motion as static and the loudness is soft.

For a diagnosis of social phobia in school settings, virtual reality content 400 may be regarded an initial or intermediate scenario.

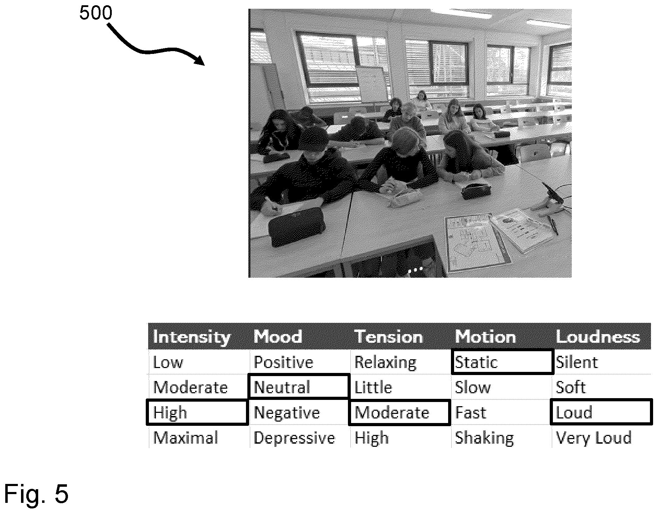

Fig. 5 illustrates a more progressed form of a social phobia in school setting using virtual reality content 500 as another example. Children begin to talk among themselves, ceasing to listen, and some displaying judgmental expressions. The intensity and noise level increase, which may lead to an amplified emotional response and heightened anxiety.

For instance, virtual reality content 500 may be regarded an endpoint of the therapy, when the user can be immersed to virtual reality content 500 without developing strong symptoms of anxiety, the therapy may be regarded successful. However, also other combinations of dimensions and levels may be the endpoint in other assessments.

The predefined therapeutic objective thus serves as a quantifiable endpoint within the multi-dimensional space of the virtual reality content, characterized by specific dimensions and levels as described.

In one implementation, the therapeutic objective can be expressed as a score within the multi-dimensional space, where progress is tracked based on how the user's feedback aligns with predefined therapeutic criteria across one or more dimensions as described.

A further embodiment involves defining the therapeutic objective as achieving the highest level in a particular dimension within the multi-dimensional space. For example, in a therapy designed to address social anxiety, the system may set a goal for the user to comfortably interact with a virtual reality scenario involving a high-stakes social situation, such as public speaking or navigating a crowded room. Progress toward this endpoint could be represented by advancing through intermediate stages, such as low-intensity scenarios (e.g., interacting with one or two individuals) to higher-intensity situations.

Another embodiment focuses on targeting the root cause of the user's problem by presenting virtual reality content specifically designed to address the core issue. This could involve looping scenes that repeatedly expose the user to a trigger until they can effectively process and manage their emotional response. For instance, a user with a fear of public speaking might be presented with progressively challenging scenarios in which they deliver a speech to increasingly larger and more interactive audiences. The looping mechanism ensures consistent exposure, enabling the user to practice coping strategies and build confidence over time.

In certain embodiments, the therapeutic objective may serve as a prerequisite for subsequent treatment stages. For example, for a user with schizophrenia who also experiences social phobia and depression, the initial therapy objective might focus on overcoming social anxiety through targeted virtual reality scenarios. Once this objective is achieved, the system could progress to addressing depressive symptoms or the user's broader challenges associated with schizophrenia. This stepwise approach ensures that therapy is tailored to the user's current capabilities while laying the groundwork for more complex interventions.

### Examples

The conditions may in one example be classified as follows, wherein the classification is of course not limited to this classification and also additional and/or less variants are feasible:

### A. Phobias - Social

Phobias related to social interactions or settings where individuals feel observed, judged, or exposed.
**1. Crowds and Gatherings**
   ∘ Variant 01: Fear of large events (concerts, conferences).
   ∘ Variant 02: Fear of everyday crowds (shopping malls, public markets).
**2. Performance Anxiety**
   o Variant 03: Fear of being evaluated or judged (public speaking, exams).
   o Variant 04: Fear of performing on stage (theater, music).
**3. Educational Environments**
   ∘ Variant 05: Anxiety in a classroom setting with peers (children, teenagers).
   ∘ Variant 06: Fear of teacher-student interactions.
**4. Interpersonal Proximity**
   ∘ Variant 07: Discomfort in close physical interactions (small meetings, personal conversations).
**5. Observational Scenarios**
   ∘ Variant 08: Anxiety when observed (e.g., eating in public, being watched at work).
**6. Interaction Anxiety**
   ∘ Variant 09: Fear of initiating or maintaining conversations.
   ∘ Variant 10: Anxiety about making eye contact.

### B. Phobias - Specific

Phobias tied to physical locations, objects, or situations that elicit fear or distress.
**1. Driving-Related**
   ∘ Variant 11: Fear of driving in urban settings (traffic congestion, city streets).
   ∘ Variant 12: Fear of driving on rural roads (dark roads, isolation).
   ∘ Variant 13: Fear of being a passenger in a vehicle.
**2. Height-Related**
   ∘ Variant 14: Fear of static heights (viewing from a tall building or tower).
   ∘ Variant 15: Fear of dynamic heights (roller coasters, climbing).
**3. Enclosed or Confined Spaces**
   ∘ Variant 16: Fear of elevators.
   ∘ Variant 17: Fear of tunnels or small rooms.
**4. Darkness and Low Visibility**
   ∘ Variant 18: Fear of dark environments (forests, basements).
   ∘ Variant 19: Fear of foggy or poorly lit areas.
**5. Medical Procedures**
   ∘ Variant 20: Fear of needles and injections.
   ∘ Variant 21: Fear of surgical or diagnostic procedures (e.g., MRI machines).
   ∘ Variant 22: Fear of dental treatments.

### C. Phobias - Animals

Phobias triggered by specific animal-related stimuli.
**1. Insects**
   ∘ Variant 23: Fear of flying insects (bees, wasps).
   ∘ Variant 24: Fear of crawling insects (spiders, cockroaches).
**2. Aquatic Creatures**
   o Variant 25: Fear of fish (e.g., swimming with fish).
   o Variant 26: Fear of worms, snails, or slugs.
**3. Reptiles and Amphibians**
   ∘ Variant 27: Fear of snakes and frogs.
   ∘ Variant 28: Fear of lizards and turtles.
**4. Birds and Mammals**
   ∘ Variant 29: Fear of large birds (e.g., pigeons, crows).
   ∘ Variant 30: Fear of domestic animals like dogs and cats.

### D. Depression - Seasonal and Bipolar Variants

Variants addressing mood disorders influenced by environmental or emotional factors.
**1. Seasonal Affective Disorder (SAD)**
   ∘ Variant 31: Depression in winter months (low sunlight exposure).
   ∘ Variant 32: Depression in summer months (intense heat, overstimulation).
**2. Emotional Contexts**
   ∘ Variant 33: Depression triggered by reflective autumn settings.
   ∘ Variant 34: Depression triggered by springtime change (allergies, transitions).
**3. Bipolar Depression**
   ∘ Variant 35: Emotional intensification through mindfulness techniques.
   ∘ Variant 36: Emotional withdrawal despite external stimulation.

### E. ADHD - Attention and Impulse Control

Conditions involving inattention, hyperactivity, or impulsivity.
**1. Inattentiveness**
   ∘ Variant 37: Difficulty focusing in noisy environments.
   ∘ Variant 38: Tendency to daydream during tasks.
**2. Hyperactivity**
   ∘ Variant 39: Inability to stay seated for long periods.
   ∘ Variant 40: Restlessness in structured environments.
**3. Impulsivity**
   ∘ Variant 41: Difficulty delaying gratification.
   ∘ Variant 42: Interruption of conversations or tasks.

### F. Substance and Behavioral Addictions

Addictions related to substances or repetitive behaviors.
**1. Substance Abuse**
   ∘ Variant 43: Alcohol dependency.
   ∘ Variant 44: Fear of reliance on controlled substances (e.g., cannabis, cocaine).
**2. Behavioral Dependencies**
   ∘ Variant 45: Addiction to gambling at casinos.
   ∘ Variant 46: Over-reliance on online betting platforms.
**3. Eating Disorders**
   ∘ Variant 47: Body dysmorphia leading to eating compulsions.
   ∘ Variant 48: Binge eating triggered by stress.

### G. Stress and Physiological Responses

Stress-related conditions and their physical manifestations.
**1. Acute Stress**
   ∘ Variant 49: Stress linked to specific events (e.g., exams, interviews).
   ∘ Variant 50: Stress caused by overwhelming work deadlines.
**2. Chronic Stress**
   ∘ Variant 51: Emotional fatigue from caregiving responsibilities.
   ∘ Variant 52: Burnout from prolonged workplace challenges.
**3. Physiological Stress**
   ∘ Variant 53: Psychosomatic symptoms (e.g., back pain, migraines).
   ∘ Variant 54: Physical health issues tied to stress (e.g., high blood pressure).

### H. Other Variants

Miscellaneous psychological health disorders, such as schizophrenia, trauma, borderline, and many more, also conditions not fitting standard categories.
**1. Self-Care**
   ∘ Variant 55: Failing to meet personal care standards.
   ∘ Variant 56: Burnout

### Example Classification of Content into Dimensions and Levels

The proposed classification framework organizes virtual reality (VR) content along five critical dimensions: intensity, mood, tension, motion, and loudness. Each dimension is further categorized into four levels, ranging from low (0) to maximal (4). This systematic approach allows for the customization and fine-tuning of VR content to elicit specific emotional and physiological responses, particularly in therapeutic applications such as the treatment of social phobia.

### 1. Intensity

∘ **Low (Level 0):** Associated with minimal sensory stimulation, promoting a sense of calm and relaxation.
∘ **Moderate (Level 1):** Provides a balanced level of engagement, suitable for scenarios requiring moderate attention.
∘ **High (Level 2):** Designed to increase alertness and engagement, often used to simulate realistic stressors.
∘ **Maximal (Level 3):** Represents overwhelming stimulation, used sparingly to replicate extreme scenarios for desensitization.

### 2. Mood

∘ **Positive (Level 0):** Encourages feelings of happiness, optimism, and safety.
∘ **Neutral (Level 1):** Provides an emotionally balanced state, neither eliciting strong positive nor negative emotions.
∘ **Negative (Level 2):** Evokes feelings of discomfort or mild stress, essential for therapeutic realism.
∘ **Depressive (Level 3):** Simulates feelings of despair or sadness, used cautiously in exposure therapy to address severe anxiety triggers.

### 3. Tension

∘ **Relaxing (Level 0):** Reduces stress and promotes relaxation.
∘ **Little (Level 1):** Introduces mild tension to maintain user engagement.
∘ **Moderate (Level 2):** Increases stress levels moderately, reflecting common social anxiety situations.
∘ **High (Level 3):** Represents significant tension to simulate highly stressful scenarios.

### 4. Motion

∘ **Static (Level 0):** Minimal movement, creating a stable and predictable environment.
∘ **Slow (Level 1):** Gradual motion to introduce variability without overwhelming the user.
∘ **Fast (Level 2):** Quick movements to heighten user awareness and mimic realistic social settings.
∘ **Shaking (Level 3):** Chaotic, unpredictable motion to replicate extreme or disorienting situations.

### 5. Loudness

∘ **Silent (Level 0):** Background noise is absent or minimal, promoting calm.
∘ **Soft (Level 1):** Subtle audio cues that enhance immersion without causing stress.
∘ **Loud (Level 2):** Elevated noise levels to simulate bustling environments or social tension.
∘ **Very Loud (Level 3):** Overwhelming audio stimuli for simulating highpressure or anxiety-inducing situations.

### Example effects on VR Content for Treating Social Phobia

The above generically described classification framework's application in social phobia therapy focuses on simulating real-life scenarios that reflect the individual's specific anxiety triggers. VR elements, such as the number of people present, social interactions, and environmental dynamics, are manipulated across the five dimensions to tailor exposure therapy effectively. Below is a detailed explanation of the social phobia-specific VR configurations:

### 1. Intensity: Number of People Present

∘ **Single Individual (Level 0):** The VR environment features one person in a static role, with predictable movements and neutral body language. This setup allows users to acclimate to basic interpersonal interactions.
∘ **Small Group (Level 1):** A few individuals are included as passive observers, exhibiting slow and predictable movements. This mimics less intimidating social situations, such as small gatherings.
∘ **Large Group (Level 2):** The VR scenario involves multiple people with dynamic expressions, posture adjustments, and varied movements. Users are exposed to frequent positional changes, which may simulate situations like attending a lecture or meeting.
∘ **Crowd (Level 3):** A densely populated environment where people move aggressively and unpredictably. This intense scenario replicates highpressure settings such as concerts or busy streets.

### 2. Proximity to Others

∘ **Distant (Level 0):** Individuals are positioned far away, with minimal emotional engagement or interaction. Body language is neutral, and movement is static, promoting a sense of safety.
∘ **Moderately Close (Level 1):** Characters may exhibit mild reactions, such as frowning, shaking their heads, or whispering. These interactions encourage users to confront mild discomfort.
∘ **Within Personal Space (Level 2):** Characters move into the user's immediate vicinity, displaying signs of frustration or anger, such as raised voices or warning gestures. This level is ideal for addressing anxiety about personal space violations.
∘ **Physical Contact (Level 3):** The most intense scenario, where characters simulate physical interactions, such as aggressive touching or shouting. This is designed to challenge extreme social phobia triggers.

### 3. Social Impact

∘ **Friendly Interaction (Level 0):** Characters smile, nod, or wave, fostering positive reinforcement and building confidence in social settings.
∘ **Neutral Observation (Level 1):** Individuals observe the user without interaction, mimicking common social experiences where users may feel judged.
∘ **Critical Reactions (Level 2):** Characters display subtle negative cues, such as frowns or whispers, simulating mild social rejection.
∘ **Hostile Behavior (Level 3):** The VR environment incorporates hostile gestures or verbal confrontations, replicating high-stakes social situations.

### 4. Movement and Interaction

∘ **Static Movement (Level 0):** Characters remain in fixed positions, allowing users to acclimate to their presence.
∘ **Predictable Movement (Level 1):** Characters exhibit slow, regular motions, creating a moderately engaging but manageable environment.
∘ **Dynamic Movement (Level 2):** Frequent positional changes and characters approaching the user simulate realistic social environments, such as crowded spaces.
∘ **Chaotic Movement (Level 3):** Rapid, unpredictable motions mimic high-stress settings, encouraging users to confront intense anxiety.

### Example correlations between variables for social phobia

The interplay between the dimensions of VR content and social phobia triggers enables highly customized therapeutic scenarios. For example:
- Low intensity, distant proximity, and static motion create a calming environment suitable for initial exposure therapy.
- Moderate intensity and neutral interactions simulate everyday social scenarios, preparing users for real-world encounters.
- High intensity, loud noise, and dynamic movement challenge users to confront significant social anxiety triggers, promoting desensitization over time.
- Maximal settings with aggressive interactions and chaotic movement are reserved for advanced therapy, addressing severe social phobia symptoms.

For instance, the predefined therapeutic objective can define a maximal setting as the therapy goal for the specific user. In other examples, the high intensity setting may be the desired objective.

The ranking among the elements of the virtual reality content in the multi-dimensional space corresponds in this example to low-intensity → moderate intensity → high intensity → maximum intensity, taking into account the combination of variables. Expressed differently, the progressively sorted ranking considers the combination between the variables / dimensions as exemplified.

This classification framework ensures that VR therapy can be personalized to meet individual needs, gradually increasing exposure to social anxiety triggers while maintaining a controlled, supportive environment.

### References

[1] https://pubmed.ncbi.nlm.nih.gov/37269086/
[2] https://www.psychiatry.org/File%20Library /Psych iatrists/Practice/DSM/APA_DSMb5_Severity-Measure-For-Social-Anxiety-Disorder-Adult.pdf
[3] https://pmc.ncbi.nlm.nih.gov/articles/PMC2792932/
[4] https://pubmed.ncbi.nlm.nih.gov/10665160/
[5] https://www.uni-marburg.de/de/fb04/team-hofmann/downloads/social_anxiety_questionnaire_for_adults_saq_psychassess.pdf
[6] https://neuroperforma.com/en/social-anxiety-test-why-is-it-important/
[7] https://www.psicothema.com/pdf/4770.pdf
[8] https://greenspacehealth.com/en-us/social-anxiety-spin/
[9] https://embrace-autism.com/the-liebowitz-social-anxiety-scale/
[10] https://www.verywellhealth.com/social-phobia-inventory-test-5271967

## Claims

1. A computer-implemented method (100) for virtual reality assisted therapy and/or differential diagnosis, comprising:
- exposing (102) a user to virtual reality content via a virtual reality headset (202) during a exposure session;
- capturing (104), via sensors, data of the user related to the exposure session virtual reality content;
- processing (106) the data using a computer-implemented algorithm to adjust the virtual reality content based on predefined therapeutic objectives.

2. The method of claim 1, wherein the predefined therapeutic objectives are determined based on responses from the user to an initial questionnaire before starting the therapy session, wherein the determination of the predefined therapeutic objectives includes in particular:
- obtaining information indicating at least one of a frequency and an intensity of a user experience related to a psychological disorder on a scale such as a Likert-type scale,
- scoring the obtained information to obtain a total score indicative of the severity of psychological disorder symptoms, in particular social anxiety symptoms, and/or one or more separate subscores for fear, anxiety and avoidance, and
- defining the therapeutic objectives to guide treatment decisions and evaluate treatment effectiveness based on the total score and/or the one or more subscores.

3. The method of claim 1 or 2, further comprising
- providing a collection of elements of virtual reality content, each element being classified as one of a plurality of levels in a multi-dimensional space comprising at least two dimensions, and
wherein the computer-implemented algorithm includes steps of:
- employing the predefined therapeutic objectives to obtain a progressively sorted ranking of the elements,
- processing the data to obtain a therapy progress decision variable and
- selecting one of the elements according to the progressively sorted ranking based on the progress decision variable to adjust the virtual reality content based on the predefined therapeutic objectives.

4. The method of claim 3, wherein the therapy progress decision variable indicates either of "up", "down" and "horizontal variation on the same level" along the progressively sorted ranking of the elements.

5. The method of claim 3 or 4, wherein the at least two dimensions include at least two of:
- intensity of the element, the intensity being preferentially defined specific to a therapeutically relevant condition;
- degree of motion,
- tension build-up,
- noise level, and
- mood.

6. The method of any of claims 3 to 5, wherein each of the dimensions of the element is classified as at least one of four levels.

7. The method of any of claims 3 to 6, wherein the progressively sorted ranking is obtained by applying a bias in the multi-dimensional space based on the predefined therapeutic objectives.

8. The method of any of claims 3 to 7, wherein at least one of the elements comprises a decision point at which one of two continuing elements associated with a different point in the multi-dimensional space, respectively, can be taken, wherein the selection of the respective continuing element is performed by processing the data of the user.

9. The method of claim 8, wherein the selection of the respective continuing element is obtained by processing at least one of biofeedback from the user and a user response to a chatbot inquiry.

10. The method of any of claims 3 to 9, wherein the adjusting the virtual reality content is performed to maximize the difficulty along the progressively sorted ranking compatible with the user's therapy progress based on the user data.

11. The method of any of the preceding claims, wherein the capturing data of the user comprises
- obtaining direct feedback after the exposure session.

12. The method of any of the preceding claims, wherein the capturing data of the user comprises at least one of:
- capturing biometric data of the user during the exposure session;
- interaction with a chatbot before, during and/or after the exposure session;
- receiving user responses to a questionnaire.

13. The method of any of the preceding claims, further comprising
- providing real-time guidance and feedback during an exposure session by a chatbot.

14. The method of any of the preceding claims, further comprising
- processing the data to identify unexpected patient reactions, and
- addressing the unexpected patient reaction using artificial intelligence.

15. An apparatus (200) for virtual reality therapy, comprising:
- a virtual reality headset (202) configured to display virtual reality content to a user;
- a plurality of sensors configured to capture biometric data of the user during the virtual reality therapy session;
- a processor configured to analyze the biometric data and dynamically adjust the virtual reality content to optimize therapeutic outcomes, wherein the processor is configured to perform the method according to any of claims 1 to 14.

16. A computer program comprising instructions which, when executed by a processor, perform the method of any of claims 1 to 14.

17. A system (200) for virtual reality therapy, comprising:
- a virtual reality display unit (202);
- a feedback control module (204, 206, 208) configured to adjust visual and auditory stimuli based on user interaction and biometric feedback;
- a storage device (210) for storing user-specific therapy data and training parameters, wherein the feedback control module is configured to operate conform the method of any of claims 1 to 14.

18. A device for facilitating virtual reality therapy, comprising:
- a display for rendering immersive virtual reality environments;
- a user interface for selecting therapy programs;
- a control unit configured to communicate with external health-monitoring devices and adapt therapy programs accordingly, wherein the control unit is configured to operate conform the method of any of claims 1 to 14.

19. Use of a virtual reality headset and a biometric feedback system in the preparation of a therapeutic method for treating psychological disorders.
